# EUROPEAN PATENT APPLICATION

(11) **EP 3 906 949 A1**
(43) Date of publication of application: **10.11.2021**
(21) Application number: 21170865.6
(22) Date of filing: 28.04.2021
(51) Int. Cl.: A61L 27/50, A61F 2/30, A61L 27/56, A61F 2/28

(54) **MEDICAL IMPLANTS WITH REINFORCING MATERIAL**

(30) Priority: 05.05.2020 US 202063020088 P
(71) Applicant: SMed-TA/TD, LLC, Columbia City, IN 46725 (US)
(72) Inventor: Kreigh, Williams R., Fort Wayne, 46804 (US)
(74) Representative: Sawodny, Michael-Wolfgang

(57) **Abstract**

A medical implant includes: an implant body including a porous, biocompatible material and being configured to allow infiltration of cells and tissues following implantation, the implant body having at least one outer surface; and a reinforcing material including at least one region of solid material having greater strength properties than the material of the implant body. The reinforcing material is affixed to the at least one outer surface to reinforce the implant body.

## Description

### Cross Reference To Related Applications

This is a non-provisional application based upon U.S. provisional patent application serial no. 63/020,088, entitled "MEDICAL IMPLANTS WITH REINFORCING MATERIAL", filed May 5, 2020, which is incorporated herein by reference.

### BACKGROUND OF THE INVENTION

### 1. Field of the Invention

The present invention relates to medical implants, and, more particularly, to medical implants including porous material.

### 2. Description of the Related Art

Medical implants, such as orthopaedic implants, are used in a variety of different applications. Many medical implants are placed in areas where the implant bears a significant amount of load from surrounding tissues, such as in orthopaedic applications. In some applications, the implant is desired to be porous to allow cell and tissue ingrowth into the implant and promote healing and implant integration. However, porosity of the implant can weaken the structure of the implant. Further, while the implant must have enough strength to bear the load, the implant cannot be made overly stiff or there is a risk that the implant will "stress shield" the surrounding bone tissue and cause bone resorption. Thus, the implant being porous to encourage cell and tissue ingrowth must be balanced with the need for the implant to have sufficient strength to bear load following implantation.

What is needed in the art is a medical implant that can be produced with a combination of properties to encourage ingrowth while maintaining sufficient strength.

### SUMMARY OF THE INVENTION

The present invention provides medical implants that have an outer surface with at least one region of reinforcing material attached thereto or otherwise integrated therein.

In some exemplary embodiments provided according to the present invention, a medical implant includes: an implant body including a porous, biocompatible material and being configured to allow infiltration of cells and tissues following implantation, the implant body having at least one outer surface; and a reinforcing material including at least one region of solid material having greater strength properties than the material of the implant body. The reinforcing material is affixed to the at least one outer surface to reinforce the implant body.

In some exemplary embodiments provided according to the present invention, a method of forming a medical implant is provided. The medical implant includes an implant body including a porous, biocompatible material and being configured to allow infiltration of cells and tissues following implantation and at least one outer surface, and a reinforcing material including at least one region of solid material having greater strength properties than the material of the implant body. The reinforcing material is affixed to the at least one outer surface to reinforce the implant body. The method includes placing the reinforcing material in contact with the material of the implant body; and affixing the reinforcing material to the material of the implant body.

An advantage of the present invention is the reinforcing material can reinforce the implant body to allow formation of cutouts in the implant body and other modifications of the implant body that weaken the implant body structure while maintaining suitable load-bearing strength.

Another advantage is the reinforcing material can be affixed to the material in a variety of ways that are economical.

### BRIEF DESCRIPTION OF THE DRAWINGS

The above-mentioned and other features and advantages of this invention, and the manner of attaining them, will become more apparent and the invention will be better understood by reference to the following description of embodiments of the invention taken in conjunction with the accompanying drawings, wherein:
FIG. 1 is a top view of an exemplary embodiment of a medical implant including an implant body and a reinforcing material provided according to the present invention;
FIG. 2 is a front view of the medical implant of FIG. 1;
FIG. 3 is a cross-sectional view of the medical implant of FIGS. 1-2 taken along line 3-3;
FIG. 4 is a top view of another exemplary embodiment of a medical implant including an implant body and a reinforcing material provided according to the present invention;
FIG. 5 is a front view of the medical implant of FIG. 4;
FIG. 6 is a cross-sectional view of the medical implant of FIGS. 4-5 taken along line 6-6;
FIG. 7 is a top view of another exemplary embodiment of a medical implant including an implant body and a reinforcing material provided according to the present invention;
FIG. 8 is a front view of the medical implant of FIG. 7;
FIG. 9 is a top view of another exemplary embodiment of a medical implant including an implant body and a reinforcing material provided according to the present invention;
FIG. 10 is a front view of the medical implant of FIG. 9;
FIG. 11 is a top view of another exemplary embodiment of a medical implant including an implant body and a reinforcing material provided according to the present invention;
FIG. 12 is a front view of the medical implant of FIG. 11; and
FIG. 13 is a flow chart illustrating an exemplary embodiment of a method of forming a medical implant provided according to the present invention.

Corresponding reference characters indicate corresponding parts throughout the several views. The exemplifications set out herein illustrate embodiments of the invention and such exemplifications are not to be construed as limiting the scope of the invention in any manner.

### DETAILED DESCRIPTION OF THE INVENTION

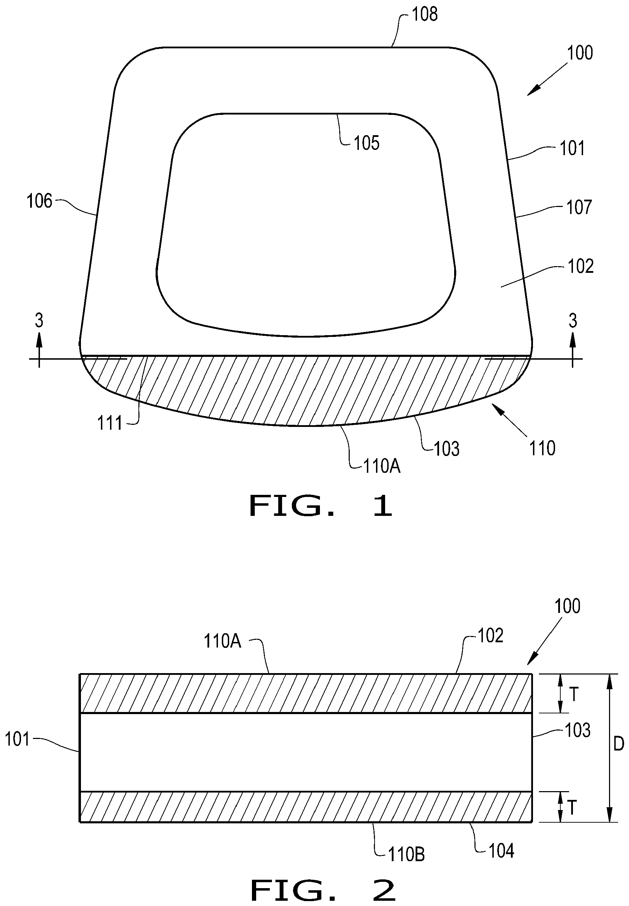

Referring now to the drawings, and more particularly to FIGS. 1-3, an exemplary embodiment of a medical implant 100, in the form of an orthopaedic implant, provided according to the present invention is illustrated. The implant 100 is illustrated in the form of an orthopaedic implant, in particular a spinal implant, but it should be appreciated that many different types of medical implants, orthopaedic or otherwise, may be provided according to the present invention. In the illustrated embodiment, the implant 100 includes an implant body 101 comprising biocompatible material, which may be a porous ingrowth material that is configured to allow infiltration and ingrowth of cells and tissues following implantation. The porous ingrowth material may comprise, but is not limited to, polymers such as polyaryl ether ketones (PAEK), e.g., polyether ether ketone (PEEK) or polyether ketone ketone (PEKK), polycarbonate urethane (PCU), and ultra-high molecular weight polyethylene (UHMWPE) as well as metals such as titanium, stainless steel, tantalum, and cobalt-chrome. The implant body 101 may incorporate a plurality of pores and have an overall porosity that is at least 20%, such as 40% to 80%. Exemplary biocompatible materials that may be used to form the implant body are commercially sold under the tradename OSTEOSYNC ® by SITES MEDICAL ® of Columbia City, Indiana. It should be appreciated that a wide variety of biocompatible materials may be used to form the implant body, and the previously described materials are exemplary only.

Many known medical implants include a variety of features, such as cutouts, to provide the implants with desired features. For example, spinal implants may include a plurality of cutouts formed in the implant body to engage one or more positioning tools and assist with positioning of the implant during a minimally invasive surgery. While such features make it easier to properly position the implant, the formed cutouts also weaken the implant body. Weakening of the implant body is especially relevant when the implant body comprises a porous material, which inevitably has decreased stiffness and other strength characteristics compared to a solid material counterpart. Thus, the ability to incorporate cutouts and other similar features in the implant body is limited by the need for the implant body to have sufficient strength to bear loads from surrounding anatomical structures during the implantation life of the implant.

To address the previously described issues with known implants, and referring still to FIGS. 1-3, the implant 100 includes at least one outer surface 102, 103, 104 with a reinforcing material 110 that is coupled to and/or integrated with the outer surface(s) 102, 103, 104 to affix the reinforcing material 110 to the implant body 101 and reinforce the implant body 101. The reinforcing material 110 may be provided as one or more regions, illustrated as two regions 110A, 110B in FIGS. 1-3, of the reinforcing material 110 that are coupled to the outer surface(s) 102, 103, 104 of the implant body. The reinforcing material 110 comprises a solid, *i.e.,* generally non-porous (no more than 10% porosity), material that has greater strength properties than the porous material of the implant body 101. For example, the reinforcing material 110 may have a greater elastic (Young's) modulus than the porous material of the implant body 101. The reinforcing material 110 may comprise, for example, metals such as titanium, cobalt-chrome, stainless steel, or tantalum, ceramics such as hydroxyapatite or calcium phosphate, and/or polymers such as high-strength thermoplastics. In some embodiments, the reinforcing material 110 is titanium or a titanium alloy that is configured to allow imaging of the region around the reinforcing material 110. The reinforcing material 110 may be formed as a plating or cladding that is bonded or otherwise affixed to the outer surface(s) 102, 103, 104 of the implant body 101. In some embodiments, each region 110A, 110B of the reinforcing material 110 has a thickness T that is approximately 5% to 50% of a top-down depth D of the implant body 101. It should be appreciated that the thickness T, and other dimensions, of the reinforcing material 110 may be altered, as desired and/or needed, to provide different reinforcing characteristics to the implant body 101.

In some embodiments, the reinforcing material 110 is bonded to the outer surface 102, 103, 104 adjacent to cutouts 105 formed in the implant body 101 or other features that tend to weaken the structure of the implant body 101. In the illustrated embodiment, for example, the reinforcing material 110 may be bonded to a top surface 102, a front surface 103, and a bottom surface 104 of the implant body 101, as can be appreciated from FIGS. 1-3. As can be seen in FIGS. 1-3, the reinforcing material 110 may cover only a portion of each of the surfaces 102, 103, 104 to which the reinforcing material 110 is bonded. The reinforcing material 110 may cover, for example, 20% to 70% of a surface area of the front surface 103, such as at least 50%, where cutouts are formed and/or will be formed. The reinforcing material 110 acts to stiffen and strengthen the implant body 101 in areas surrounding the reinforcing material 110, allowing a greater number of cutouts or other features to be formed in the implant 100 without lowering the strength of the implant body 101 to a point where the implant body 101 is unable to safely bear loads following implantation. It should be appreciated that while the reinforcing material 110 is illustrated and described as being bonded to the front surface 103 and top and bottom surfaces 102, 104 of the implant body 101, the reinforcing material 110 may also be bonded, alternatively or additionally, to one or more lateral surfaces 106, 107 of the implant body 101 and/or a rear surface 108 of the implant body 101, especially if cutouts or other features are formed in one or more of these surfaces 106, 107, 108.

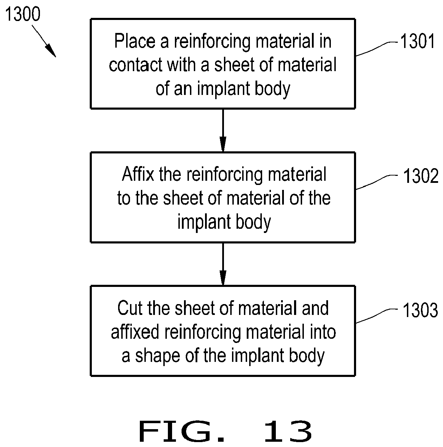

To form the implant 100, and referring still to FIGS. 1-3 and now to FIG. 13 as well, a method 1300 of forming the implant 100 is provided that includes placing 1301 the reinforcing material 110 in contact with the material of the implant body 101 and affixing 1302 the reinforcing material 110 to the material of the implant body 101. The material of the implant body 101, which may be porous, may be provided as a sheet of material. The sheet of material may have a greater width (lateral-to-lateral surface dimension) and depth (front-to-back dimension) than the final implant body 101, but have a thickness (top-to-bottom dimension) that is equal, or similar, to the thickness of the final implant body 101. The sheet of material may be formed by bonding together a plurality of thinner plates of material to form the sheet. The reinforcing material 110, which may be in the form of a strip of solid titanium, is placed 1301 in contact with the sheet of material and affixed 1302 to the sheet of material in the desired location(s). The reinforcing material 110 may be affixed to the sheet of material by, for example, heat bonding, e.g., diffusion bonding, the reinforcing material 110 to the sheet of material and/or mechanically affixing the reinforcing material 110 to the sheet of material, as will be described further herein. As illustrated especially in FIG. 1, affixing the strip of reinforcing material 110 to the sheet of material results in a straight surface 111 where the reinforcing material 110 meets the biocompatible material of the sheet. Before and/or after affixing the reinforcing material 110 to the sheet of material, surfaces of the reinforcing material 110 and/or the sheet of material may be finished by, e.g., polishing and/or chemical treatment.

The sheet of material and affixed reinforcing material 110 may be cut into smaller strips of material each having a reduced width and depth compared to the sheet, but a similar thickness. In some embodiments, cutouts and/or other features are machined or otherwise formed into a strip of material at defined locations that will correspond to the locations of the feature(s) in the final implant body 101 produced from the strip of material. The bonded strip of material and reinforcing material is cut 1303 into the final shape of the implant body 101, such as by wire cutting, to form the implant 100 having the implant body 101 comprising the material of the strip of material and the affixed reinforcing material 110. Thus, implants 100 provided according to the present invention may be formed in an economical manner by forming several implants from one original sheet of material to reduce the amount of time it takes to form each individual implant.

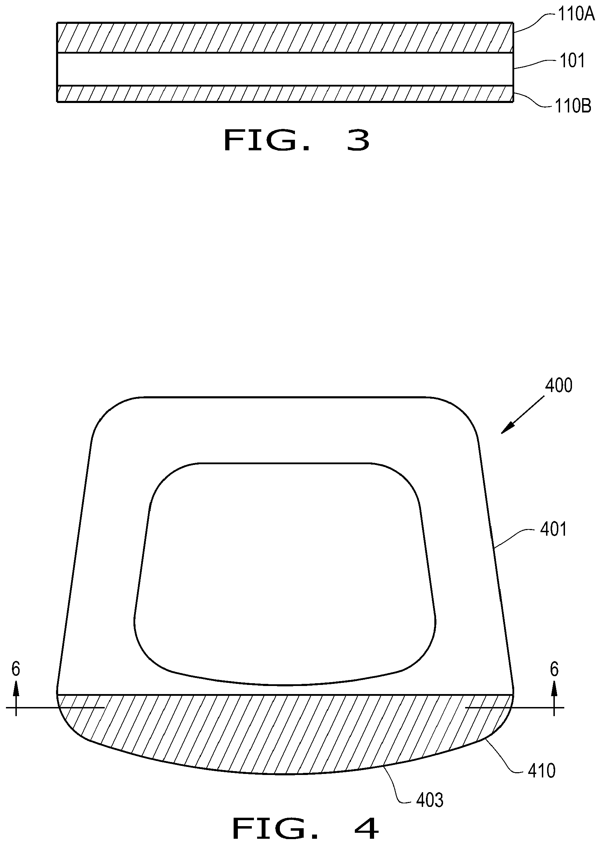

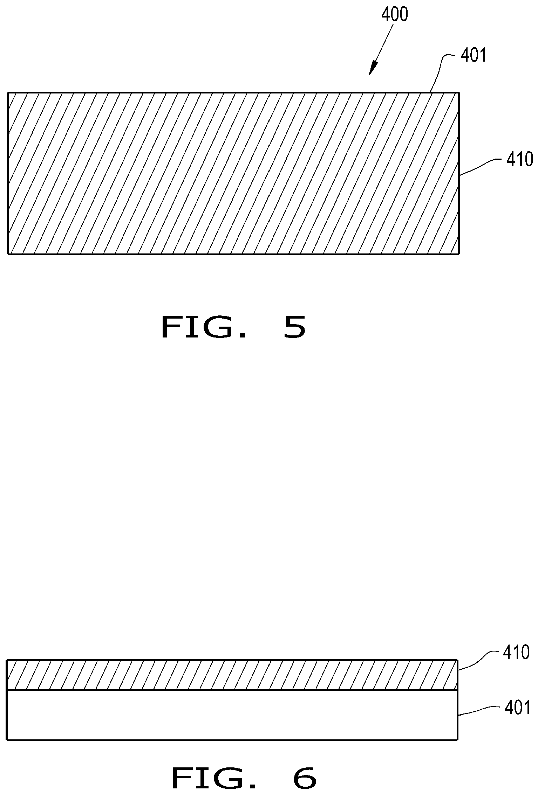

Referring now to FIGS. 4-6, another exemplary embodiment of an orthopaedic implant 400 provided according to the present invention is illustrated. The orthopaedic implant 400 of FIGS. 4-6 is similar to the orthopaedic implant 100 of FIGS. 1-3 in that the orthopaedic implant 400 includes an implant body 401 comprising a biocompatible material with a reinforcing material 410 affixed thereto. The primary difference between the orthopaedic implant 400 of FIGS. 4-6 and the orthopaedic implant 100 of FIGS. 1-3 is that the implant body 401 has a front surface 403 that is entirely covered by affixed reinforcing material 410, which may be a strip of solid titanium as previously described. The reinforcing material 410 may be affixed to a surface of the strip of material so the reinforcing material 410 will form the front surface 403 of the final formed implant. In other respects, the orthopaedic implant 400 of FIGS. 4-6 may be similar to the previously described orthopaedic implant 100 of FIGS. 1-3.

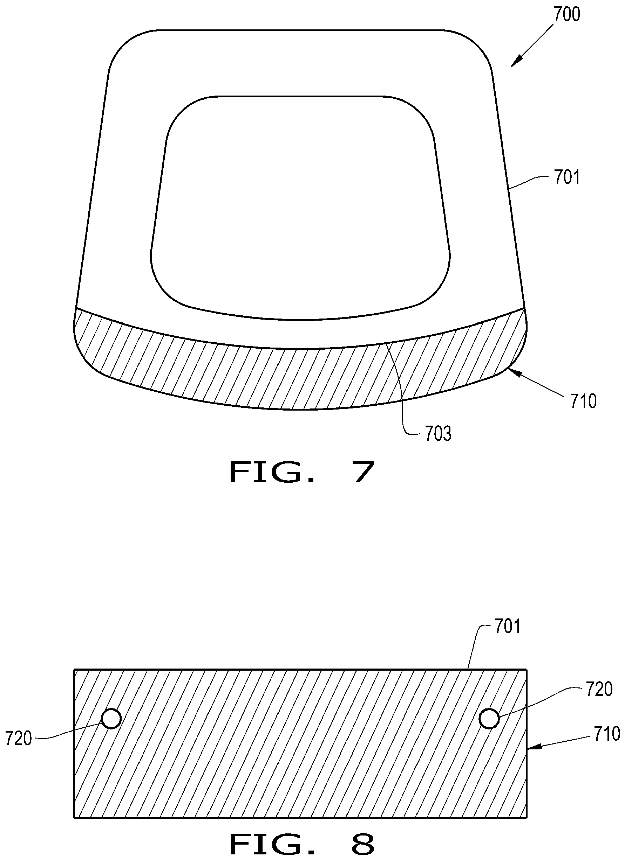

In some embodiments, and referring now to FIGS. 7 and 8, an exemplary embodiment of an orthopaedic implant 700 is provided that includes an implant body 701 and a reinforcing material 710 affixed to the implant body 701. The orthopaedic implant 700 may be similar to the previously described orthopaedic implant 400 of FIGS. 4-6 but differ in that the orthopaedic implant 700 of FIGS. 7 and 8 is formed by affixing the reinforcing material 710 to the implant body 701 after the general shape of the implant body 701 has been formed. For example, the reinforcing material 710 may be affixed to the implant body 701 after the general shape of the implant body 701 is cut from a sheet of material, rather than affixing the reinforcing material 710 to the sheet of material prior to cutting the general shape of the implant body 701. As can be appreciated from FIG. 7, the implant body 701 has a curved surface 703, which may define a shape of a front surface of the formed implant, to which the reinforcing material 710 is affixed. The reinforcing material 710 may be formed as a strip having a complementary shape to the curved surface 703 of the implant body 701 prior to affixing the reinforcing material 710 to the implant body 701. Alternatively, the reinforcing material 710 may be affixed to the implant body 701 and then machined or otherwise processed to shape the reinforcing material 710 and form the final shape of the orthopaedic implant 700. It should thus be appreciated that the shape of the reinforcing material 710 affixed to the implant body 701 may be adjusted in many different ways according to the present invention.

In some embodiments, the reinforcing material 110, 410, 710 is mechanically affixed to the implant body 101, 401, 701 to form the orthopaedic implant 100, 400, 700. The reinforcing material 110, 410, 710 may be mechanically affixed to the implant body 101, 401, 701 in a variety of ways, including but not limited to by fasteners 720 (illustrated in FIG. 7), such as screws, or by an interference fit formed between the reinforcing material 110, 410, 710 and the implant body 101, 401, 701. In some embodiments, the reinforcing material 110, 410, 710 is both mechanically affixed to the implant body 101, 401, 701 and also heat bonded to the implant body 101, 401, 701 by, e.g., diffusion bonding to connect the reinforcing material 110, 410, 710 and the implant body 101, 401, 701. It should thus be appreciated that the reinforcing material 110, 410, 710 may be affixed to the implant body 101, 401, 701 in many different ways according to the present invention.

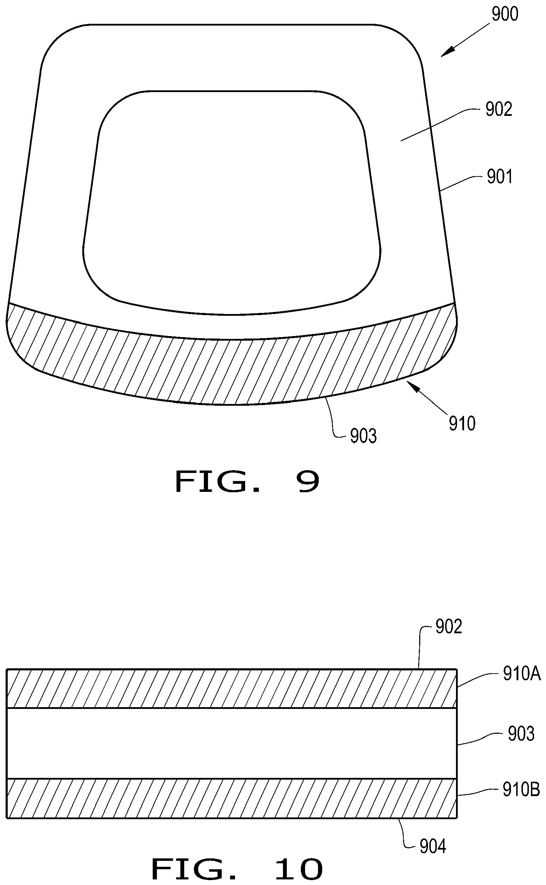

Referring now to FIGS. 9 and 10, another exemplary embodiment of an orthopaedic implant 900 including an implant body 901 and a reinforcing material 910 provided according to the present invention is illustrated. The orthopaedic implant 900 illustrated in FIGS. 9 and 10 is similar to the orthopaedic implant 100 of FIGS. 1-3 in that two distinct regions 910A, 910B of reinforcing material 910 are affixed to the implant body 901. Both regions 910A, 910B of reinforcing material 910 are affixed to a front surface 903 of the implant body 903, with one of the regions 910B being additionally affixed to a bottom surface 904 of the implant body 901 and the other region 910A being additionally affixed to a top surface 902 of the implant body 901. Similarly to the orthopaedic implant 700 of FIGS. 7 and 8, the reinforcing material regions 910A, 910B may each be both heat bonded to the implant body 901 as well as mechanically affixed to the implant body 901, using fasteners or otherwise. In other respects, the orthopaedic implant 900 of FIGS. 9 and 10 can be similar to previously described orthopaedic implants 100, 400, 700.

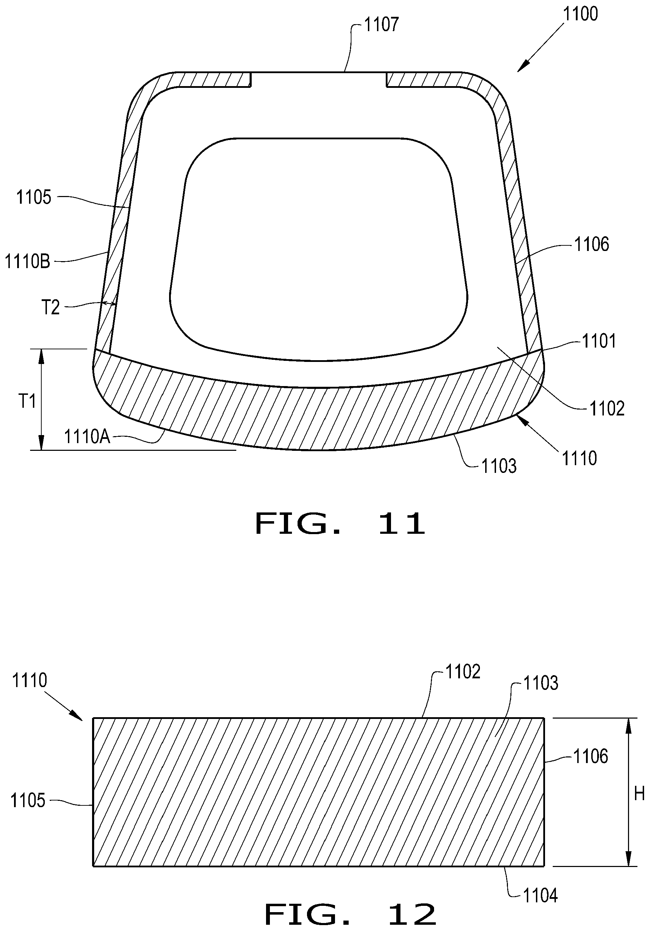

Referring now to FIGS. 11 and 12, another exemplary embodiment of an orthopaedic implant 1100 is illustrated that includes an implant body 1101 and a reinforcing material 1110 affixed to the implant body 1101. The reinforcing material 1110 may be affixed to a plurality of outer surfaces 1102, 1103, 1104, 1105, 1106, 1107 of the implant body so the reinforcing material 1110 substantially covers a front surface 1103 and lateral surfaces 1105, 1106 of the implant body 1101 while also covering a portion of a rear surface 1107 of the implant body 1101. In this respect, the reinforcing material 1110 may act as an outer shell that is affixed to porous material of the implant body 1101 to provide rigidity and strength to the orthopaedic implant 1100. In some embodiments, the reinforcing material 1110 may have multiple thicknesses. For example, the reinforcing material 1110 may have a first thickness T1 that is equal to a height H of the implant body 1101 in a first region 1110A of the reinforcing material 1110 that is affixed to the front surface 1103 and a second thickness T2 that is considerably less, such as 10% to 30% of the height H of the implant body 1101, in a second region 1110B of the reinforcing material 1110 that is affixed to one or more of the lateral surfaces 1105, 1106 and/or the rear surface 1107. In this respect, the reinforcing material shell 1110 may be thicker in the first region 1110A affixed to the front surface 1103, where greater strength is required, while being thinner in the second region 1110B affixed to one or more of the other surfaces 1105, 1106, 1107, where less strength may be required. The reinforcing material 1110 may be affixed to the implant body 1101 using heat bonding and/or mechanical fixation, as previously described, to stably affix the greater amount of reinforcing material 1110 to the implant body 1101. The reinforcing material 1110 may be affixed to the implant body 1101 after the general shape of the implant body 1101 has been cut from a sheet of material, as previously described. In other respects, the orthopaedic implant 1100 of FIGS. 11 and 12 may be similar to previously described orthopaedic implants 100, 400, 700, 900.

While this invention has been described with respect to at least one embodiment, the present invention can be further modified within the spirit and scope of this disclosure. This application is therefore intended to cover any variations, uses, or adaptations of the invention using its general principles. Further, this application is intended to cover such departures from the present disclosure as come within known or customary practice in the art to which this invention pertains and which fall within the limits of the appended claims.

The application comprises aspects which are disclosed in the sentences below, which are part of the description but not claims in accordance with J 15/88 of the Boards of appeal.

### SENTENCES

1. A medical implant, comprising:
   an implant body comprising a porous, biocompatible material and being configured to allow infiltration of cells and tissues following implantation, the implant body comprising at least one outer surface; and
   a reinforcing material comprising at least one region of solid material having greater strength properties than the material of the implant body, the reinforcing material being affixed to the at least one outer surface to reinforce the implant body.
2. The medical implant of sentence 1, wherein the implant body defines a top-down depth and the reinforcing material defines a thickness that is 5% to 50% of the top-down depth of the implant body.
3. The medical implant of sentence 1, wherein the reinforcing material comprises a first portion and a second portion, the first portion defining a first thickness and the second portion defining a second thickness that is less than the first thickness.
4. The medical implant of sentence 3, wherein the implant body defines a height and the first thickness is equal to the height of the implant body.
5. The medical implant of sentence 4, wherein the second thickness is 10% to 30% of the height of the implant body.
6. The medical implant of sentence 3, wherein the at least one outer surface comprises a front surface and at least one lateral surface, the first portion being affixed to the front surface and the second portion being affixed to the at least one lateral surface.
7. The medical implant of sentence 6, wherein the first portion covers the front surface and the second portion covers the at least one lateral surface.
8. The medical implant of sentence 1, wherein the material of the implant body has a porosity of at least 20%.
9. The medical implant of sentence 1, wherein the reinforcing material comprises a different material than the material of the implant body.
10. The medical implant of sentence 1, wherein the implant body has at least one cutout formed therein.
11. The medical implant of sentence 1, wherein the reinforcing material has a greater elastic modulus than the material of the implant body.
12. The medical implant of sentence 1, wherein the reinforcing material covers the at least one outer surface.
13. The medical implant of sentence 1, wherein the reinforcing material is affixed to the at least one outer surface through at least one of heat bonding or mechanical fixation.
14. The medical implant of sentence 1, wherein the at least one region comprises a first region and a second region that is spaced from the first region.
15. The medical implant of sentence 1, wherein the reinforcing material covers 20% to 70% of a surface area of the at least one outer surface to which the reinforcing material is affixed.
16. The medical implant of sentence 1, wherein the reinforcing material comprises at least one of titanium, cobalt-chrome, stainless steel, tantalum, hydroxyapatite, calcium phosphate, or a polymer.
17. The medical implant of sentence 16, wherein the reinforcing material comprises titanium.
18. A method of forming a medical implant, the medical implant comprising an implant body comprising a porous, biocompatible material and being configured to allow infiltration of cells and tissues following implantation and at least one outer surface, and a reinforcing material comprising at least one region of solid material having greater strength properties than the material of the implant body, the reinforcing material being affixed to the at least one outer surface to reinforce the implant body, the method comprising:
   placing the reinforcing material in contact with the material of the implant body; and
   affixing the reinforcing material to the material of the implant body.
19. The method of sentence 18, wherein the material of the implant body is in the form of a sheet of material and the reinforcing material is affixed to the sheet of material, the method further comprising cutting the sheet of material and affixed reinforcing material into a shape of the implant body.
20. The method of sentence 18, wherein the reinforcing material is in the form of a strip of material.

## Claims

1. A medical implant, comprising:
an implant body comprising a porous, biocompatible material and being configured to allow infiltration of cells and tissues following implantation, the implant body comprising at least one outer surface; and
a reinforcing material comprising at least one region of solid material having greater strength properties than the material of the implant body, the reinforcing material being affixed to the at least one outer surface to reinforce the implant body.

2. The medical implant of claim 1, wherein the implant body defines a top-down depth and the reinforcing material defines a thickness that is 5% to 50% of the top-down depth of the implant body.

3. The medical implant of at least one of the claims 1 to 2, wherein the reinforcing material comprises a first portion and a second portion, the first portion defining a first thickness, preferably equal to the height of the implant body and the second portion defining a second thickness, preferably 10% to 30% of the height of the implant body that is less than the first thickness.

4. The medical implant of claim 3, wherein the at least one outer surface comprises a front surface and at least one lateral surface, the first portion being affixed to the front surface and the second portion being affixed to the at least one lateral surface.

5. The medical implant of at least one of the claims 1 to 4, wherein the first portion covers the front surface and the second portion covers the at least one lateral surface.

6. The medical implant of at least one of the claims 1 to 5, wherein the material of the implant body has a porosity of at least 20% and/or wherein the reinforcing material comprises a different material than the material of the implant body.

7. The medical implant of at least one of the claims 1 to 6, wherein the implant body has at least one cutout formed therein and/or wherein the reinforcing material has a greater elastic modulus than the material of the implant body.

8. The medical implant of at least one of the claims 1 to 7, wherein the reinforcing material covers the at least one outer surface.

9. The medical implant of at least one of the claims 1 to 8, wherein the reinforcing material is affixed to the at least one outer surface through at least one of heat bonding or mechanical fixation.

10. The medical implant of at least one of the claims 1 to 9, wherein the at least one region comprises a first region and a second region that is spaced from the first region.

11. The medical implant of at least one of the claims 1 to 10, wherein the reinforcing material covers 20% to 70% of a surface area of the at least one outer surface to which the reinforcing material is affixed.

12. The medical implant of at least one of the claims 1 to 11, wherein the reinforcing material comprises at least one of titanium, cobalt-chrome, stainless steel, tantalum, hydroxyapatite, calcium phosphate, or a polymer.

13. A method of forming a medical implant, the medical implant comprising an implant body comprising a porous, biocompatible material and being configured to allow infiltration of cells and tissues following implantation and at least one outer surface, and a reinforcing material comprising at least one region of solid material having greater strength properties than the material of the implant body, the reinforcing material being affixed to the at least one outer surface to reinforce the implant body, the method comprising:
placing the reinforcing material in contact with the material of the implant body; and
affixing the reinforcing material to the material of the implant body.

14. The method of claim 13, wherein the material of the implant body is in the form of a sheet of material and the reinforcing material is affixed to the sheet of material, the method further comprising cutting the sheet of material and affixed reinforcing material into a shape of the implant body.

15. The method of at least one of the claims 13 to 14, wherein the reinforcing material is in the form of a strip of material.
